(19) Europäisches Patentamt
European Patent Office
Office européen des brevets

(11) Numéro de publication: **0 069 001**
**B1**

# (12) FASCICULE DE BREVET EUROPEEN

(45) Date de publication du fascicule du brevet: **17.04.85**

(51) Int. Cl.⁴: **C 07 D 495/04** // A61K31/435 ,(C07D495/04, 333:00, 221:00)

(21) Numéro de dépôt: **82401131.6**

(22) Date de dépôt: **21.06.82**

(54) **Procédé de préparation de dérivés de la tétrahydro-5,6,7,7a 4H-thiéno (3,2-C) pyridinone-2.**

(30) Priorité **30.06.81 FR 8113067**

(43) Date de publication de la demande:
**05.01.83 Bulletin 83/1**

(45) Mention de la délivrance du brevet:
**17.04.85 Bulletin 85/16**

(84) Etats contractants désignés:
**AT BE CH DE FR GB IT LI LU NL SE**

(56) Documents cités
**EP - A - 0 053 950**
**FR - A - 2 319 642**

(73) Titulaire: **SANOFI, Société dite:, 40, Avenue George V, F-75008 Paris (FR)**

(72) Inventeur: **Bousquet, André, 27 Rue de la Vigne, F-04200 Sisteron (FR)**
Inventeur: **Heymes, Alain, Chemin de l'Adrech, F-04200 Sisteron (FR)**

(74) Mandataire: **Bressand, Georges et al, c/o CABINET LAVOIX 2 Place d'Estienne d'Orves, F-75441 Paris Cedex 09 (FR)**

**EP 0 069 001 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention est relative à un nouveau procédé de préparation de dérivés de la tétrahydro-5,6,7,7a 4H-thiéno (3,2-c) pyridinone-2 répondant à la formule I

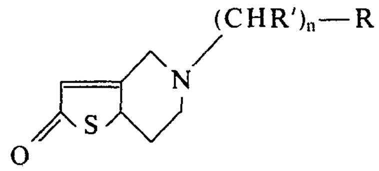

(I)

dans laquelle R représente un atome d'hydrogène ou un radical phényle éventuellement substitué par au moins un atome d'halogène ou un groupement alcoyle inférieur, alcoxy inférieur, nitro, carboxy, alcoxycarbonyle, R' représente un atome d'hydrogène ou un groupe alcoyle inférieur et n est 0 ou un nombre entier de 1 à 4.

Ces composés qui présentent des propriétés anti-agrégantes plaquettaires et anti-thrombotiques ont fait l'objet pour leur application thérapeutique, de trois demandes de brevet français (8 025 274, 8 025 275 et 8 025 276) de la part de la présente demanderesse.

Cependant les procédés décrits dans les brevets référencés ne permettent pas d'obtenir, sur le plan industriel, des rendements très élevés.

La présente invention a pour but de fournir un procédé simple, facilement mis en oeuvre et d'un rendement global supérieur au regard des techniques antérieures d'obtention des composés de formule I. Il est rappelé que, dans le brevet français n° 7 523 786, il est décrit un procédé d'obtention de dérivés de la thiénopyridine de formule II

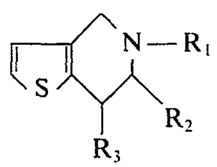

(II)

dans laquelle $R_1$ représente un radical alcoyle, aryle ou aralcoyle, éventuellement substitué et $R_2$ et $R_3$ représentent un atome d'hydrogène ou un radical alcoyle inférieur, aryle ou hétérocyclique, par cyclisation en deux stades de composés de formule III

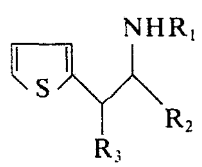

(III)

et que, par ailleurs, dans le brevet français n° 8 025 276, on décrit la transformation de dérives de formule IV

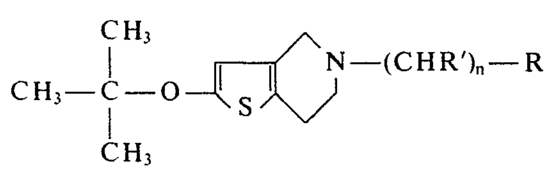

(IV)

dans laquelle R', R et n sont tels que définis pour la formule I, en composés de formule I »par chauffage à des températures comprises entre 80 et 180°C en présence d'un acide minéral ou organique«.

La demanderesse a trouve, et tel est l'objet de la présente invention, qu'en appliquant les conditions d'obtention des dérivés de formule II à partir des composés de formule III aux dérivés de formule V

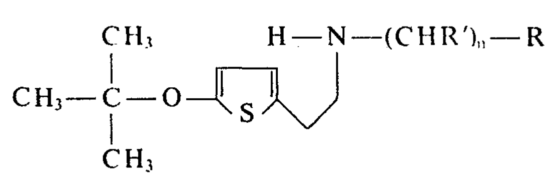

(V)

dans laquelle R, R' et n sont tels que définis ci-dessus, on accédait de manière inattendue, directement, dans des conditions moins contraignantes, notamment sur le plan de la température et avec des rendements satisfaisant, aux composés de formule I.

Conformément au prodédé de l'invention, pour accéder aux composés de formule I:

a) on met à réagir sous forte agitation, à la température ambiante, un dérivé de formule V avec du formol en milieu aqueux pour obtenir un composé de réaction dont on peut présumer que la structure est celle de la formule VI

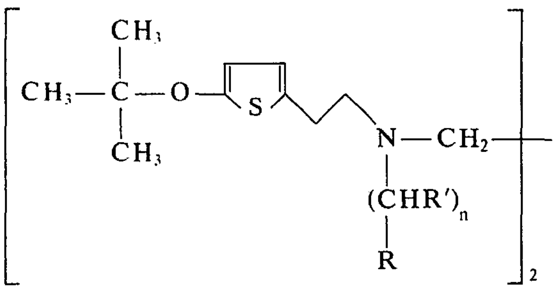

(VI)

b) composé qui est traité dans un second temps avec une solution d'acide chlorhydrique sec dans un solvant aprotique polaire.

De manière plus précise, il est essentiel que le produit d'addition du formaldéhyde (dont on met en oeuvre un à cinq équivalents sur le plen stoechiométrique) sur le composé de formule V soit isolé et obtenu exempt d'eau. Il peut cependant être utilisé dans l'étape suivante en solution dans un solvant inerte tel qu'un hydrocarbure aromatique comme le benzène ou le toluène ou un solvant halogéné comme le chlorure de méthylène ou tout autre solvant semblable compatible avec la nature du produit, le point essentiel étant qu'une telle solution soit anhydre.

Le produit obtenu à l'étape a) est ajouté à une solution d'acide chlorhydrique sec dans un solvant polaire, aprotique, de préférence le diméthylformamide. On peut également utiliser d'autres solvants de nature semblable comme le diméthylsulfoxide, la N-méthyl pyrrolidone ou le N,N-diméthylacétamide.

En général, l'acide chlorhydrique est utilisé en équivalence stoechiométrique mais peut être en excès jusqu'à 100 % par rapport à cette équivalence. On opère généralement entre −20 et 50°C et plus particulièrement entre 0 et 30°C.

Les composés de formule I ainsi obtenus peuvent ensuite être isolés et purifiés selon les méthodes habituelles. Pour réaliser ces opérations, il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

L'exemple suivant illustre l'invention sans la limiter.

## Exemple

### Préparation du chlorhydrate de (chloro-2 benzyl)-5 tétrahydro-5,6,7,7a 4H-thiéno (3,2-c) pyridinone-2

a) on additonne, sous forte agitation, et en maintenant la température à 20°C, 12,94 g (0,04 mole) de N ortho-chlorobenzyl [terbutoxy-5-(thiényl-2)]-2 éthylamine à 14 g (0,16 mole) d'une solution aqueuse à 35 % (p/p) de formaldéhyde. L'addition terminée, on maintient le mileu réactionnel sous agitation durant 15 minutes puis on y ajoute 15 ml de chlorure de méthylène. La phase organique est iscleé, lavée avec une solution aqueuse de chlorure de sodium puis séchée sur sulfate de sodium.

b) la solution ainsi obtenue est additionnée sous agitation et en maintenant la température comprise entre 20 et 25°C à 8,25 ml d'une solution 4,85 N d'acide chlorhydrique gazeux dans la diméthylformamide. Après 20 minutes d'agitation supplémentaires, le milieu réactionnel est additionné de 48 ml d'une solution aqueuse N de bicarbonate de sodium. La phase organique est isolée puis évaporée sous vide à température inférieure à 60°C. L'huile résiduelle est concrétisée par trituration dans 20 ml d'éthanol.

On obtient ainsi après filtration et séchage 5,8 g de (chloro-2 benzyl)-5 tétrahydro-5,6,7,7a 4H-thiéno (3,2-c) pyridinone-2 (rendement = 52 %) sous forme de cristaux.

F = 73°C
R.M.N. ($CDCl_3$): 7,1−7,6 (m, 4H); 6,2 (s, 1H);
4,2−4,7 (m, 1H); 3,9 (s, 2H);
1,5−4,2 (m, 6H).
Chlorhydrate hémihydrate: F = 180°C (dec.).

**Revendications**

1. Prodédé de préparation de dérivés de la tétrahydro-5,6,7,7a-4H-thiéno (3,2-c) pyridinone-2 de formule I:

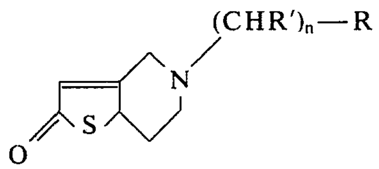

(I)

dans laquelle R représente un atome d'hydrogène ou un radical phényle éventuellement substitué par au moins un atome d'halogène ou un groupement alcoyle inférieur, alcoxy inférieur, nitro, carboxy, alcoxy carbonyle; R' représente un atome d'hydrogène ou un groupe alcoyle inférieur et n est 0 ou un nombre entier de 1 à 4, caractérisé en ce qu'on met à réagir sous forte agitation, à la température ambiante, un composé de formule V:

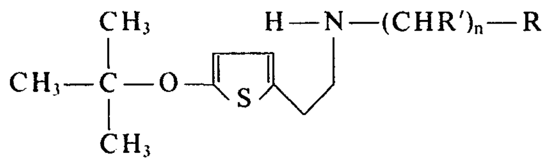

(V)

avec du formol H—CHO en milieu aqueux, pour obtenir un composé de formule VI:

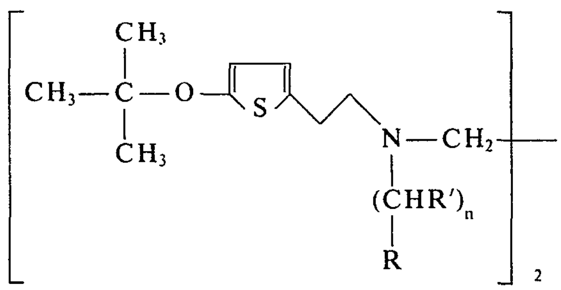

(VI)

que l'on isole et sèche, puis on traite par de l'acide chlorhydrique sec dans un solvant polaire aprotique pour obtenir un dérivé de formule I.

2. Procédé selon la revendication 1, caractérisé en ce que le composé de formule VI est dissous dans un solvant organique inerte avant la réaction avec l'acide chlorhydrique sec.

3. Procédé suivant la revendication 1, caractérisé en ce que le traitement du composé VI par l'acide chlorhydrique est effectué en milieu anhydre, à une température de −20 à +50°C.

4. Procédé selon la revendication 3, caractérisé en ce que le traitement par l'acide chlorhydrique est effectué à une température de 0 à 30°C.

5. Procédé suivant la revendication 1, caractérisé en que le solvant polaire aprotique est le diméthylformamide, le diméthylsulfoxyde, la N-méthyl pyrrolidone ou le N, N-diméthylacétamide.

**Patentansprüche**

1. Verfahren zur Herstellung von Derivaten von 5,6,7,7a-Tetrahydro-4-H-thieno(3,2-c)pyridin-2-on der Formel I

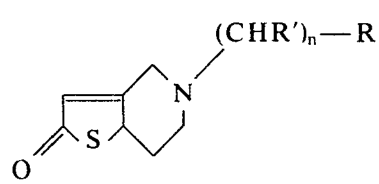

(I)

in der R ein Wasserstoffatom oder ein gegebenenfalls mit wenigstens einem Halogenatom substituierter Phenylrest oder ein niederer Alkylrest, ein niederer Alkoxyrest, eine Nitrogruppe, Carboxylgruppe,

Alkoxycarbonylgruppe ist, R' ein Wasserstoffatom oder ein niederer Alkylrest ist und n für 0 oder eine ganze Zahl von 1 bis 4 steht, dadurch gekennzeichnet, daß man unter starkem Rühren bei Umgebungstemperatur eine Verbindung der Formel V

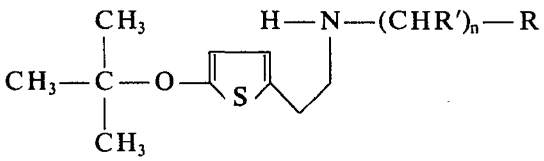

(V)

mit der Formel H—CHO in wäßrigem Medium umsetzt, um eine Verbindung der Formel VI

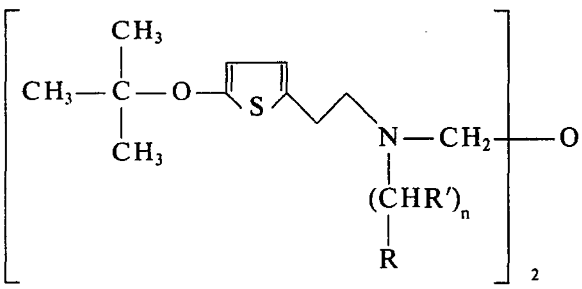

(VI)

zu erhalten, die man isoliert und trocknet und dann mit trockener Salzsäure in einem aprotischen polaren Lösungsmittel behandelt, um ein Derivat der Formel I zu erhalten.

2. Verfahren nach Anspruch 1, dadurch gekennzeichnet, daß die Verbindung der Formel VI vor der Umsetzung mit trockener Salzsäure in einem inerten organischen Lösungsmittel gelöst wird.

3. Verfahren nach Anspruch 1, dadurch gekennzeichnet, daß die Behandlung der Verbindung VI mit Salzsäure in wasserfreiem Medium bei einer Temperatur von −20 bis +50°C durchgeführt wird.

4. Verfahren nach Anspruch 3, dadurch gekennzeichnet, daß die Behandlung mit Salzsäure bei einer Temperatur von 0 bis 30°C durchgeführt wird.

5. Verfahren nach Anspruch 1, dadurch gekennzeichnet, daß das aprotische polare Lösungsmittel Dimethylformamid, Dimethylsulfoxid, N-Methylpyrrolidon oder N,N-Dimethylacetamid ist.

**Claims**

1. Process for the preparation of 5,6,7,7a-tetrahydro-4H-thieno(3,2-c)pyridin-2-one derivatives of the formula:

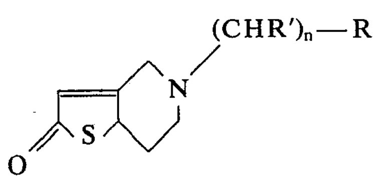

(I)

in which R represents a hydrogen atom or a phenyl radical optionally substituted with at least one halogen atom or a lower alkyl, lower alkoxy, nitro, carboxy, alkoxycarbonyl group; R' represents a hydrogen atom or a lower alkyl group an n is 0 or an integer from 1—4, comprising reacting a compound of the formula:

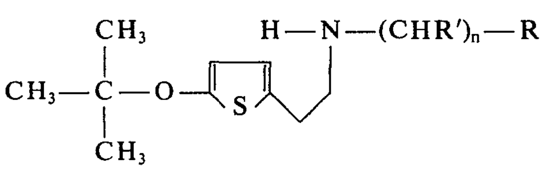

(V)

with formaldehyde H—CHO in aqueous medium, to give a compound of the formula:

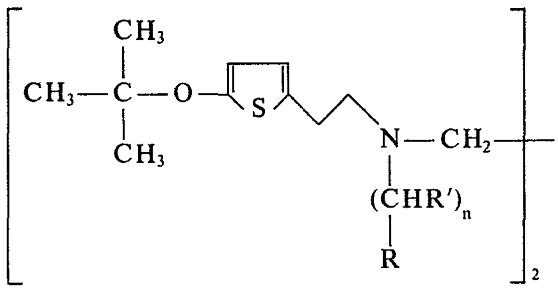

(VI)

which is isolated and dried, then treating with dry hydrochloric acid in an aprotic polar solvent, to give a derivate of the formula (I).

2. Process as claimed in claim 1, characterized in that the compound of formula (VI) is dissolved in an inert organic solvent before the reaction with dry hydrochloric acid.

3. Process as claimed in claim 1, characterized in that the treatment of compound (VI) with hydrochloric acid is carried out in anhydrous medium, at a temperature of −20 to +50°C.

4. Process as claimed in claim 3, characterized in that the treatment with hydrochloric acid is carried out at a temperature of 0 to 30°C.

5. Process a claimed in claim 1, characterized in that the aprotic polar solvent is dimethylformamide, dimethylsulfoxide, N-methylpyrrolidone or N,N-dimethylacetamide.